# EUROPEAN PATENT SPECIFICATION

(11) **EP 3 171 905 B1**
(45) Date of publication and mention of the grant of the patent: **12.12.2018**
(21) Application number: 15730808.1
(22) Date of filing: 24.06.2015
(51) Int. Cl.: A61L 31/02, A61L 31/08, A61L 31/10, A61L 31/14, A61L 31/16

(54) **BIODEGRADABLE METAL STENT AND METHODS**
BIOLOGISCH ABBAUBARER METALL-STENT UND VERFAHREN
STENT MÉTALLIQUE BIODÉGRADABLE ET PROCÉDÉS

(30) Priority: 22.07.2014 US 201462027253 P; 22.07.2014 EP 14177945
(43) Date of publication of application: 31.05.2017
(73) Proprietor: Biotronik AG, 8180 Bülach (CH)
(72) Inventor: SAVAGE, Douglas R., Del Mar, California 92014 (US); NGUYEN, John Dang, San Diego, California 92129 (US)
(74) Representative: Randoll, Sören
(86) International application number: PCT/EP2015/064186
(87) International publication number: WO 2016/012179

(56) References cited:
- EP-A2- 2 422 826
- US-A1- 2007 281 117
- US-A1- 2009 030 507

## Description

The present invention relates generally to a biodegradable metal stent for maintaining patency of an animal body vessel, and methods of making and using the stent.

Stents are endovascular prostheses that may be used to treat occlusions in animal vascular lumens. Stents are generally tubular in configuration and are open at both longitudinal ends. The prostheses are placed into the body in a collapsed geometry, usually into a vessel lumen, and then expanded into a dilated configuration when at the treatment site. Such stents have been established as a preferred method of treatment for vascular diseases which occur in the coronary and peripheral vasculature.

When appropriately placed, stents can be used to establish an appropriate widening of a vascular lumen to permit proper vascular lumen flow which is the primary goal of this therapy. The presence of this foreign body may however then initiate one or more of several biological responses which may have undesired effects. A normal response to vascular stent placement is the healing sequence which begins with platelet activation and fibrin formation to the eventual replacement of fibrin with smooth muscle cells, effectively encapsulating the stent with the formation of neointima. This process of healing vascular remodeling is beneficial to form a layer of natural tissue which surrounds the implant. However, an undesired effect of stenting is creation of sufficient neointima so as to re-occlude the diseased vessel, necessitating additional vascular therapy. The presence of a permanent stent will alter the dynamics of native vessel motion. In a static condition the vessel sections supported by the rigid stent are restrained while the adjacent sections are subject to increased motion. By thus altering the motion of the native vessel with a permanent implant it is not uncommon to observe large restenosis areas in the vessel lumen in the location of the ends of the stent. It would be therefore desirable to utilize a vascular prosthesis which would provide necessary support to a dilated occlusion until adequate vascular healing is complete and then would biodegrade in a manner which is non-inflammatory to the tissue surrounding vessel tissue.

Traditional vascular prostheses are comprised of durable materials such as stainless steel, nickel titanium alloys, and cobalt chromium alloys which are designed to maintain their structural integrity for the entire life of the animal or human recipient. Biodegradable materials are available as substitutes for these durable materials. Bioerodable polymers, for example, can be used to fabricate vascular prostheses with sufficient strength on deployment to overcome radial forces associated with the acute closure of diseased vessels. Igaki et. al. (U.S. Pat. No. 6,045,568) have disclosed a luminal stent formed by knitting a sole yarn of bioresorbable polymer fiber, such as a fiber of polylactic acid. This device, upon expansion, may retain its shape for several weeks inside a vessel. Kaplan (U.S. Pat. No. 5,342,348) discloses polymer stents which dilate body lumens and also release bioactive substances which are useful for preventing restenosis. Biodegradable stents constructed from polymers will begin to degrade upon expansion in a body lumen when exposed to aqueous body fluids. The degradation may occur through bulk hydrolysis or through surface erosion. Polymer prosthesis may not provide optimum performance in accurately dilating diseased vessels due to the inherent elastic recoil of the polymer materials used for their construction. Essentially, the stent is expanded from a compressed condition to a larger diameter which is desired to maintain the vessel lumen in an open condition. The stent must afford a sufficient carrying strength to hold the vessel open for a period of time to allow for revascularization of the lumen tissues.

In order to avoid unnecessary vessel damage it is also desirable that, after expansion and after removal of the balloon, the stent only slightly springs back (recoils) in order to have to expand the stent upon expansion thereof only as little as possible beyond the desired final diameter. Thus, a higher recoil character would require greater vessel expansion and subsequent potential undesirable vessel damage. Metal materials which have been considered for the purpose of creating endoprostheses include magnesium, iron, and aluminum. These base elements may be alloyed with rare earth and other metals. Unlike polymers, metals may be selected to minimize the elastic recoil. Metals also may be modified to through alloying, heat treatment, and cold working techniques to provide greater strength which will result in increased vessel radial support. For this reason biodegradable metals have been utilized as a material alternative to polymers to construct endovascular prostheses. Heublein (U.S. Pat. No. 7,879,367 and German patents) describes a medical implant made of a metallic magnesium material. After fulfilling its temporary support function, the implant is degraded by corrosion at a predetermined rate. Atanososka (U.S. Pat. No. 7,998,192) describes an endoprostheses comprising a body defining a flow passage, the body capable of maintaining patency in a blood vessel, the body comprising iron or an alloy of iron, with further a nano-structured surface comprising iron oxide. Unlike permanent metal implants, those fabricated from unprotected biodegradable metals may begin to corrode shortly after expansion in the body. Electrolyte incursion into the metallic structure will result in oxidation of the metal. Gradual oxidation weakens the structure, and this weakening is further accelerated by motion-induced fatigue due to bending, pulsatile activity, torsion, and stretching of the device.

To control the rate of degradation of the expanded endoprosthesis it may be necessary to provide protection to the bare metallic structure with one or more barrier means. Organic or inorganic surface coatings may be used to provide a barrier between the bare metal surface of the implant and corrosive body fluids. Those skilled in the art will appreciate that whatever treatment which is applied to the implant surface should be chosen so that the implant remains fully biodegradable, even with the application of the treatment. As such, the bioerodable endoprosthesis with biostable inorganic layer described by Weber (U.S. Pat. No. 8,128,689) could result in a biodegradable implant device with a permanent remnant of applied surface treatment after implant in an animal body and after subsequent erosion of the underlying stent structure. Kappelt (U.S. Pub. No. 2008/0243242) describes a method for producing a corrosion-inhibiting coating on an implant made of a biocorrodible magnesium alloy, the method comprising providing the implant, and treating the implant surface using an aqueous or alcoholic conversion solution containing various ions. Kurze (U.S. Pub. No. 2012/0156477) details a method of protecting magnesium by placement of a device of that metal into a reactor vessel with humid carbon dioxide to produce a coating comprised of magnesium carbonate. Those who are familiar with metallurgy know that an inorganic coating may be created on the surface of a metal stent by oxidizing the surface with hot gases in air or in a controlled environment. Anodizing and electroplating methods can also be used, along with immersion or spray coating of specialized barrier paints and coverings, lacquering, enameling, and vapor deposition methods. In light of the desire to maintain a fully biodegradable implant the particular inorganic coating should metabolize in a finite period of time along with the bioerodable implant substrate. The corrosion-inhibiting surface treatments described should cause a temporary inhibition, but not a complete suppression, of the corrosion of the device in a physiological environment. Because the endoprosthesis is subject to the forces related to vascular dynamics, whatever inorganic coating that might be applied should not interfere with the ability of the implant to flex within its natural physiological environment.

Biodegradable polymers may also be used to create a corrosion barrier to a biodegradable substrate device. These polymers may be cast, spray coated, overmolded, or applied by dip coating onto the surfaces to be protected. They may be applied to the entire surface or only to portions of the surface where protection is desired. Such biodegradable medical polymers are found currently in orthopedic applications such as bone screws and plates. They also are found in microparticle drug delivery products for treatment of cancer and other human disorders. Often an antiproliferative or immunosuppressive drug will be mixed with the polymer in the form of a drug coating an placed onto some or all of the surfaces of the vascular implant in order to eliminate or moderate the continued growth of neointimal smooth muscle cells, the precursor to vascular restenosis. Other therapeutic drugs including antibiotics, steroids, growth factors, anti-clotting compounds may also be used. Representative examples of polymers that may be used to coat a bioabsorbable endoprosthesis include, but are not limited to poly(caprolactone), polyvinyl esters, poly(D,L lactic acid), poly(L-lactide), poly(lactid-co-glycolide), polyorthoester, polyanhydride, poly(trimethyliene carbonate), polyurethanes, silicones, oils, and fatty acids. To secure the polymer to the substrate an adhesion promoter such as a liquid silane formulation might be applied prior to the application of the polymer. Gale (U.S. Pat. No. 8,172,897) describes a device and a method of manufacturing an implantable device wherein a biodegradable polymer region contacts a pure or nearly pure metallic region. In one embodiment a mixture of polymer and bioerodable metal particles is used to coat the device. Wilcox (U.S. Pub. No. 2009/0240323) also notes that a polymer may be applied to an implant with a magnesium core to provide a specific degradation time for the core structure.

EP 2422826 A2, US 2009/0030507 A1 and US 2007/0281117 A1 disclose stents having at least one coating.

An endoprosthesis, once expanded in an animal body vessel, is subjected to forces related to the geometry and dynamics of vascular motion. Contraction of the heart myocardium during the cardiac cycle will, for example, generate bending motion, torsion, extension and contraction of the implant in-situ. Further, pulsatile motion in the form of radial pulsation of the vessel will cause repeated increases and decreases in the implant diameter. The combination of physiological forces results in localized strain areas which can occur throughout the body of the implant. To prevent fracture and subsequent incursion of physiological fluids any corrosion barriers applied to the implant would require that the barriers be sufficiently elastic or thin in thickness to overcome dynamic fatigue caused by repeated strain. Inorganic barrier layers such as oxides can be inherently brittle and tend to crack and form fissures that extend into the underlying substrate when exposed to repeated bending, torsion, or lateral displacement. When expanded and placed into a physiological solution, electrolyte ingress into these fissures will begin to invade and corrode the underlying substrate material. It is therefore beneficial to place an additional barrier layer which is very elastic over the first organic or inorganic barrier that will serve to seal and protect any of the fissures which may expose the underlying structure. Such combination of barrier layers must be sufficiently thick to retard corrosion of the implant and yet must not promote excessive inflammation and granuloma reaction within the vessel tissues which surround the implanted device.

The single addition of an inorganic barrier layer to the metallic surface may generally not be sufficient to alone prevent premature corrosion because of cracks which appear in the inherently brittle barrier material during clinical use. The addition of a single thicker elastic barrier layer to the metallic surface may not be sufficient to alone prevent premature corrosion because in many the cases the elastic barrier layer may imbibe physiological fluids and be hydrated, causing subsequent fluid electrolyte ingress to the metallic surface of the implant. A single thick elastic barrier layer such as a biodegradable polymer is undesirable for other reasons. A thick barrier layer will add in dimension to the overall diameter of the implant when it is applied to delivery balloon or system. A larger diameter mounted implant can be more difficult to deliver to an affected vessel by a clinical specialist. Also, a greater inflammatory response may result from using a thicker biodegradable polymer layer as macrophages and cellular components infiltrate and metabolize the degrading material. The optimum corrosion protection for a biodegradable metallic prosthesis is thus derived using at least a thin elastic organic layer or multiple protective layers as described herein.

It is suggested an expandable prosthesis for vascular placement comprising or consisting of a tubular biodegradable metal structure, a biodegradable organic layer covering the surfaces of the structure and optionally a biodegradable polymer coating covering the organic surface layer. The biodegradable organic layer is a siloxane layer of 20 nanometers to 5 micrometer and is applied by injection of monomer vapor into a chamber with an applied plasma.

The invention includes an expandable prosthesis, e.g., stent as a tubular biodegradable metal structure with a base metal structure. The tubular biodegradable metal structure can be formed from suitable biodegradable metals such as magnesium, iron, zinc, aluminum or the like as well as of an alloy of these metals containing a majority by weight percentage of one of the above elements and combined with other trace metals.

In one example, the invention includes an expandable prosthesis, e.g., stent with a base metal structure comprised of an alloy of magnesium containing a majority by weight percentage of the element magnesium and combined with other trace metals. One representative material is alloy ZRE1 from Magnesium Elektron UK. This material is an alloy of magnesium containing zinc, zirconium, and rare earth metals. The endoprosthesis is formed from a laser cut tube of the alloy and is subsequently electropolished to a desired geometry.

In a first experiment, one not according to the invention, the device is then placed into a Thermo-Scientific Inc. model FD1545M furnace which has been heated to 426 degrees C for approximately eight minutes and then removed. An oxide protective layer has now formed on the device surface which has a scaled appearance with a visible boundary surrounding each scale when viewed in a scanning electron microscope at 250X magnification. An alternative surface treatment, one according to an embodiment of the invention, has also been applied to create a thin protective layer achieved by placing the device into a chamber with an applied plasma with an inert gas such as Argon or Nitrogen and injecting at least one organosilicon compound, e.g. the liquid organosilicon compound hexamethyldisiloxane (HMDSO), into the chamber to achieve a uniform and thin coating of units and combinations of dimethylsiloxane. A similar compound, divinyltetramethyldisiloxane (DVTMDSO) will yield a more flexible thin layer of units and combinations of dimethylsiloxane. Glass-like coatings with a composition of SiOx can also be applied in plasma chamber, but are not according to the invention. Yet another similar deposition process may be used to create a very thin layer of diamond-like coating (DLC) comprised of carbon element, which is also not according to the invention.

A biodegradable polymer coating is then formed over the entire surface of the device. The polymer can be one of several known to those skilled in the art. A representative example is polycaprolactone PC-12 from Purac of the Netherlands. Poly(L-lactide), poly(d,l lactide), trimethylene carbonate, poly(glycolide) and blends and copolymers thereof may be suitable for the purpose of creating a biodegradable coating with good sealing properties. Further examples for the polymers are described herein below. The additional polymer layer will preferably have an elongation at break of greater than fifteen percent and a tensile strength of at least 20Mpa. The polymer material choice and the thickness of the polymer coating should be engineered to provide a desired degradation time for the useful service life of the implant. The polymer has been dissolved in an appropriate solvent such as acetone, chloroform, dichloromethane or other suitable chemical. The ideal solvent will be almost entirely removed in a subsequent drying step using applied vacuum and heat. A therapeutic drug may be present in this coating layer depending on the clinical application of the device. The coating formulation can be applied by spray coating, dip coating, casting, vapor deposition, manual application with a syringe, or other means. In the current example a 120 kHz Micromist nozzle from Sono-tek Inc. is used to deposit a coating of between 10 and 35 microns depth of polymer coating onto all surfaces of the device.

At the fabricator's discretion a drug-containing layer may be then coated onto the surface of the endoprosthesis. The drug may be dissolved into a solvent which is then applied directly to the implant surface wherein the drug will ingress at least partially into the previously applied polymer coating. The drug may also be incorporated into a coating formulation with a polymer where the ratio of drug to polymer is about five percent to about ninety percent depending on the specific polymer used and also the method of application.

To evaluate one aspect of the invention three separate coating sample sets were fabricated. The first sample set included magnesium stents that were 20mm in length and were mounted onto balloon delivery catheters. The second sample set included magnesium stents that were coated by using a spray method with polycaprolactone polymer formulation. These samples were then dried in a vacuum oven at a temperature of 50 deg. C and a vacuum level of 27 inHg for a period of 48 hours. The third set of samples was processed like the second set. However, an additional heat treat process was applied to the bare metal stents before application of the polymer by placing the stent samples into furnace preheated to 426 deg. C with ambient oxygen for a period of five minutes.

Before application of the polycaprolactone polymer measurements of the surface of the unprocessed and processed stents were performed with Auger analysis using a PHI model 600 spectroscopy system with 3 kilovolts applied voltage. Those stents which were not heat treated revealed a minimal oxide layer and those with the heat treatment revealed an oxide layer with surface depth of magnesium oxide of approximately 400 Angstroms.

All three sample sets were deployed into silicone tubes with dimensions and physical attributes which would correlate to coronary arteries. The tubes were filled with porcine serum through a heated pumping circuit. The silicone tubes were repeatedly bent at an angle and movement cycle corresponding to a human left anterior descending artery. The implants were visually observed at regular intervals and their appearance was recorded digitally.

The stent samples were maintained cycling in the test apparatus for thirty days, a period of time representing one month of life in a human coronary artery. At the end of this time period the tubes were removed from the apparatus and flushed with deionized water. The vessels with the stents still inside were placed into a test jig and crushed with an Imada model 25 compression tester with a load cell rated at 25 Newtons compressive force.

Those stents which were not treated were broken and did not retain any residual radial force after the first week of testing. The group of stents which were treated with polycaprolactone polymer only retained approximately 2.3 Newtons radial force required to compress the deployed stents to approximately 80% of their deployed diameter. The group of stents which were treated with an initial heat treatment prior to coating with polycaprolactone polymer retained approximately 2.9 Newtons radial force required to compress the deployed stents to approximately 80% of their deployed diameter. The addition of the heat treat step was indicated as responsible for increasing the radial force of stents in this in-vitro test by approximately 26% compared to those stents which were not heat treated.

In another example of the invention magnesium stents were first immersed in 1 molar concentration sodium bicarbonate solution at room temperature for a period of three minutes. At this time the surface of the stents was chemically oxidized from a primary magnesium metal alloy content to magnesium hydroxide. The stents were then rinsed in deionized water for one minute to remove residual electrolyte and then dried. They were then placed in an insulated furnace which was preheated to 426 deg. C for five minutes and then removed to cool to room temperature. In the heat treatment process the stent surface magnesium hydroxide material was converted to magnesium oxide and water vapor. Subsequent measurement of the surface of the processed stents with Auger analysis using a PHI model 600 spectroscopy system with 3 kilovolts applied voltage revealed a minimum surface depth of magnesium oxide of 160 Angstroms.

To test the capability of a magnesium oxide surface coating to protect the stent in a corrosive environment the coated samples were placed into glass vials filled with phosphate buffered saline as were samples of untreated stents. The vials were maintained at room temperature. The samples in each container were visually monitored for evidence of corrosion. After one day gas bubbles had formed on the surface of the untreated stents and the stent were visibly tarnished. After two weeks the untreated stents had corroded through the metal in numerous locations on the stent. However, after two weeks the treated stents did not show any visible tarnish, bubbling, or material erosion, indicating that the treatment had provided surface corrosion protection.

In a third example of the invention stents twenty millimeters in length and fabricated from magnesium alloy tubes were coated with a surface treatment or thin layer of a siloxane applied using a plasma enhanced chemical vapor deposition process. Siloxane materials, including polydimethylsiloxane (PDMS) and other organosilane compounds are preferred as protective layers because they may be applied uniformly in a plasma process and have a greater degree of ductility when applied as compared to inorganic oxides such as silicon dioxide or as compared to diamond-like carbon coatings. The ductility or flexibility of the siloxanes can be modified or adjusted by varying process parameters which include applied plasma power, the nature of the siloxane precursor, the gas to fluid ratios, time, chamber pressure, and the injection rate. Siloxane materials are also hydrophobic and thus provide an effective barrier for the base metal of the implant device to surrounding fluid electrolytes. Thin layers of siloxane may biodegrade over time, especially in an alkali or base aqueous environment. A Mettler MT5 precision balance was used to determine an average coating layer weight for each stent of approximately 60 micrograms, measuring the weight both before and after the coating deposition.

Subsequent measurement of the surface of the processed stents with Auger analysis using a PHI model 600 spectroscopy system with 3 kilovolts applied voltage revealed a surface depth of the applied coating of approximately three hundred nanometers.

A group of the siloxane coated stents and a group of non- siloxane coated stents were further coated with an additional layer of polycaprolactone biodegradable polymer with a weight of 4 milligrams after drying in a vacuum chamber set to 27 inches of mercury vacuum and 50 degree C for 48 hours and then weighed to determine the weight of the coating material. Yet an additional drug layer of 160 milligrams of polylactic acid polymer and 160 milligrams of the drug rapamycin was applied to both groups. Both stent polymer coatings were applied using a Sono-tek Inc. ultrasonic spray nozzle which directed the spray cone perpendicular to a stent mounted on a mandrel appropriately sized to the inner diameter of the stent. After the coating process was completed the stents were dried in a vacuum chamber set to 27 inches of mercury vacuum and at room temperature for 48 hours and then weighed to determine the weight of the drug coating material.

Stents from three individual groups were mounted to 22 millimeter angioplasty balloons using a Blockwise Engineering model RMD crimping device. The groups were of the following constructions. The first group was made of magnesium stents coated only with siloxane surface treatment. The second group was made of magnesium stents coated only with polycaprolactone polymer and mixed rapamycin and PLA drug layer. The third group was made of magnesium stents coated with siloxane with an additional coating of polycaprolactone and with the additional mixed rapamycin and PLA drug layer. All of the mounted stents were individually expanded to 18 ATM with a B. Braun indeflation device filled with deionized water into silicone tubes with diameter compliance of 3-5% per 100mm Hg applied pressure. After deployment the stents were evaluated through the transparent tubes using an optical microscope at 20X magnification for cracks in the coating which might have resulted from ductile movement of the stressed zones, particularly in areas of the inner crown region of the stent struts. The tubes were filled with porcine serum through a heated pumping circuit. The silicone tubes were repeatedly bent at an angle and movement cycle corresponding to a human left anterior descending artery. The implants were visually observed at regular intervals and their appearance was recorded digitally.

The stent samples were maintained cycling in the test apparatus for thirty days, a period of time representing one month of life in a human coronary artery. At the end of this time period the tubes were removed from the apparatus and flushed with deionized water. The vessels with the stents still inside were placed into a test jig and crushed with an Imada model 25 compression tester with a load cell rated at 25 Newtons compressive force. The group of stents which were treated with the thin surface treatment layer maintained a radial force of approximately 2.0N required to compress the deployed stent to approximately 80% of their deployed diameter. The group of stents which were treated with the biodegradable polymer layer maintained a radial force of approximately 3.0N required to compress the deployed stents to approximately 80% of their deployed diameter. The group of stents which were treated with the thin surface treatment layer and the biodegradable polymer maintained a radial force of approximately 4.5N required to compress the deployed stent to approximately 80% of their deployed diameter. Accordingly, it was determined that the combination of both the thin surface treatment layer and the thicker biodegradable polymer layer was responsible for maintaining optimum protection of the base metal from corrosive effects of the porcine plasma over a period of 30 days.

For the present invention the process of plasma application of thin layer is be suitably modified to obtained desired mechanical and surface properties. A thin layer coating might be optimized for adhesion, moisture barrier properties, surface hardness, or flexibility. The liquid monomer material from which vapor is used to initiate the layer can be chosen from simple organic solvents, fluorinated organic compounds or organosilicon compounds. Also gaseous monomer material can be used such as methane or ethylene. In a preferred embodiment the liquid monomer is chosen from organic solvents and organosilicon compounds, and is chosen most preferred from organosilicon compounds. Suitable organic solvents/compounds can be selected from compounds such as benzene, toluene-2,4-diisocyante, 2-pyrollidone, tetramethyltin, vinyl acetate, styrene, acetylene and p-toluidine. Suitable organosilicon compounds include hexamethyldisiloxane, divinyltetramethyldisiloxane, methyltrimethoxysilane, tetramethylsiloxane, hexamethyldisiloxane, tetramethylsilane, tetramethoxysilane. The monomers mentioned above may be used alone or in combination to achieve desired properties. The amount of the applied layer should be optimized to provide adequate thickness and the lowest residual stress.

According to the present invention the biodegradable organic layer comprises or consists of an organic compound applied by injection of monomer vapor into a chamber with an applied plasma. The monomer vapor is created from the monomer compounds described in the preceding paragraph. The biodegradable organic layer comprises or consists of a siloxane layer, applied by injection of monomer vapor into a chamber with an applied plasma, wherein the monomer vapor is preferably created from at least one of the following compounds hexamethyldisiloxane, divinyltetramethyldisiloxane, methyltrimethoxysilane, tetramethylsiloxane, hexamethyldisiloxane, tetramethylsilane and tetramethoxysilane. In a further preferred embodiment of the invention the biodegradable organic layer is a uniform polymethylsiloxane layer applied by injection of HMDSO (hexamethyldisiloxane) or DVTMDSO (divinyltetramethyldisiloxane) vapor into a chamber with an applied plasma. In a further preferred embodiment of the invention the biodegradable organic layer is a uniform polymethylsiloxane layer applied by injection of HMDSO (hexamethyldisiloxane) vapor into a chamber with an applied plasma. Yet in another preferred embodiment the biodegradable organic layer is a uniform polymethylsiloxane layer applied by injection of DVTMDSO (divinyltetramethyldisiloxane) vapor into a chamber with an applied plasma.
For applying a biodegradable organic layer plasma enhanced CVD is especially advantageous in comparison to other used methods such as or high temperature CVDs or immersion in a chemical bath because it can occur at very gentle conditions such as low temperatures and in a "dry" process that disfavors corrosion on the surface of the endoprothesis. As a result the underlying material base material of the endoprosthesis is unchanged. Also, many of the use precursors would not survive harsh conditions such as very high temperatures and would decompose before the proper use in the plasma and would thereby form irregular, carbonaceous layers not capable of providing the advantages described herein. Additionally with organic or organosilicon presursors as stated herein PECVD provides a uniform thickness and can be applied to all of the surfaces of complex geometries, like stents. PECVD further creates very dense and continuous layers which totally encapsulate the underlying endoprosthesis like a stent. Moreover, the use of organic or organosilicon precursors with plasma creates coatings which have greater elasticity in comparison when provided by inorganic precursors and/or other harsher methods. Such great elasticity can be enhanced when a layer of biodegradable polymer is applied on the biodegradable organic protection layer.
An endoprosthesis comprising a biodegradable organic layer covering the surfaces of the structure and a biodegradable polymer coating covering the organic surface layer is particularly capable of not only maintaining optimum protection of the base metal, but also due to the enhanced elasticity of the protective layers of greatly withstanding the forces present in an pulsating vascular environment.

An additional effort was made to obtain adequate corrosion protection on a magnesium stent with a reduced mass of biodegradable polymer applied to the device. Stents twenty millimeters in length and fabricated from magnesium alloy tubes were coated with a surface treatment or thin layer of an organosiloxane derived from plasma deposition of 1,3 divinyltetramethylsiloxane (DVTMDSO) vapor and Argon carrier gas applied using a plasma enhanced chemical vapor deposition process. The thin layer derived from the DVTMDSO monomer is known to be more flexible and deposits on the substrate more rapidly than the thin layer derived from HMDSO monomer. In this evaluation the stents were first cleaned with Argon plasma at a pressure of 0.3mBar in a 40 Khz plasma chamber with an applied power of approximately 200 Watts for ten minutes. After cleaning, liquid DVTMDSO monomer at a temperature of 23 degrees C was vaporized at a pressure of 0.6mBar and a flow rate of 200cm³ /minute and introduced into the plasma chamber with an applied power output of 100 Watts. The layer was applied to the stents for 90 minutes and the process was then terminated. A Mettler MT5 precision balance was used to determine an average coating layer weight for each stent of approximately 600 micrograms, measuring the weight both before and after the coating deposition.

A group of the stents with DVTMDSO-derived thin layer coating stents was divided, and one half of the group were further coated with an additional layer of polycaprolactone biodegradable polymer with a weight of just 2 milligrams after drying in a vacuum chamber set to 27 inches of mercury vacuum and 50 degree C for 48 hours and then weighed to determine the weight of the coating material. Yet an additional drug layer of 160 milligrams of polylactic acid polymer and 160 milligrams of the drug rapamycin was applied to both groups. Both stent polymer coatings were applied using a Sono-tek Inc. ultrasonic spray nozzle which directed the spray cone perpendicular to a stent mounted on a mandrel appropriately sized to the inner diameter of the stent. After the coating process was completed the stents were dried in a vacuum chamber set to 27 inches of mercury vacuum and at room temperature for 48 hours and then weighed to determine the weight of the polycaprolactone and drug coating materials.

As in prior evaluations the stents from both groups were mounted to 22 millimeter angioplasty balloons using a Blockwise Engineering model RMD crimping device. All of the mounted stents were individually expanded to 18 ATM with a B. Braun indeflation device filled with deionized water into silicone tubes with diameter compliance of 3-5% per 100mm Hg applied pressure. The tubes were filled with porcine serum through a heated pumping circuit. The silicone tubes were repeatedly bent at an angle and movement cycle corresponding to a human left anterior descending artery. The implants were visually observed at regular intervals and their appearance was recorded digitally.

At the end of the 14 day time period the tubes were removed from the apparatus and flushed with deionized water. The vessels with the stents still inside were evaluated under an Olympus model SZ30 optical microscope The group of stents which were treated with the thin surface treatment layer and drug/polymer layer were observed to have an average of 8 fractures occurring over the entire 20mm length of the device. The group of stents which were treated with the thin surface treatment layer, biodegradable polymer layer, and drug/polymer layer were observed to have an average of 2 fractures occurring over the entire 20mm length of the device. Although the mass of the thin layer DVTMDSO-derived layer was dramatically increased, the combination of both the thin surface treatment layer and the thicker biodegradable polymer layer was responsible for maintaining optimum protection of the base metal from corrosive effects of the porcine plasma.

It is important to note that simply increasing the thickness of a single surface treatment layer on the base metal, or by increasing the thickness of a single biodegradable polymer layer on the base metal an optimum protection may not be achieved. In an embodiment of the invention wherein the thin surface treatment layer is sufficiently flexible that cracks or fissures will not be created in this thin layer during clinical use of the device a subsequent biodegradable polymer layer may not be required. Such sufficient protection can be derived when the thickness of the biodegradable organic layer ranges from about 20 nanometers to about 5 microns. The additional application of a biodegradable polymer may for instance be required to prevent incursion of fluid electrolytes into the base metal of the stent and initiation of the corrosion process. In such case the endoprosthesis is beneficially provided with additional elasticity and corrosion protection.
- Fig. 1: shows a vascular endoprosthesis in accordance with the present invention prior to mounting on a balloon catheter
- Fig. 2: illustrates a cross-section of a single filament of an endovascular prosthesis with a primary surface protective layer and an additional polymer passivation coating.
- Fig. 3: illustrates a cross-section of a single filament of an endovascular prosthesis with a primary surface protective layer and an additional polymer passivation coating with a yet additional drug-containing coating layer.
- Fig. 4: is a schematic representation of the fabrication sequence to generate multiple protective layers noted in an embodiment of the current invention.
- Fig. 5: is a schematic representation of the plasma enhanced chemical vapor deposition (PECVD) coating onto a surface.
- Fig. 6: shows a means of application of a uniform coating layer of polymer passivation material or drug or drug and polymer combination.
- Fig. 7: shows a scanning electron microscope image of a single curved portion of a filament of an endovascular prosthesis comprised of magnesium.
- Fig. 8: shows a scanning electron microscope image of a single curved portion of a filament of an endovascular prosthesis comprised of magnesium which has an applied surface protection layer which occurs at the outer boundary of the cross section.
- Fig. 9: shows a scanning electron microscope image of a single curved portion of a filament of an endovascular prosthesis comprised of magnesium which has an inherent surface protection layer which occurs at the outer boundary of the cross section.
- Fig. 10: shows a scanning electron microscope image of a single curved portion of a filament of an endovascular prosthesis comprised of magnesium which has an applied surface protection layer and a polymer passivation layer and a drug eluting polymer layer in accordance with the present invention.
- Fig. 11: shows surface elemental analysis of an endovascular prosthesis comprised of a magnesium alloy.
- Fig. 12: shows surface elemental analysis of an endovascular prosthesis comprised of a magnesium alloy which has been treated with heat in a controlled environment to produce an inherent surface oxide layer.
- Fig. 13: shows surface elemental analysis of an endovascular prosthesis comprised of a magnesium alloy which has been treated a plasma enhanced chemical vapor deposition process to produce an applied surface protection layer with silicon and oxygen in combination.
- Fig. 14: shows surface elemental analysis of an endovascular prosthesis comprised of a magnesium alloy which has been treated a plasma enhanced chemical vapor deposition process to produce an applied surface protection layer with carbon, silicon and oxygen in combination.
- Fig. 15: shows an angiography image of the left anterior descending artery of a porcine heart in which is deployed an endoprosthesis which has been processed in accordance with methods described in the present invention which is produced after the endoprosthesis has been deployed within the heart for a period of twenty-eight days.
- Fig. 16: shows an X-ray image of an endoprosthesis comprised of magnesium with other materials and processed in a manner that does not provide adequate protection to the base material which is provided twenty-eight days after the endoprosthesis has been deployed into a coronary arter of an animal heart.
- Fig. 17: shows an X-ray image of an endoprosthesis comprised of magnesium and processed in a manner consistent with methods and processes specified in the current invention to provide adequate protection to the base material which is provided twenty-eight days after the endoprosthesis has been deployed into a coronary artery of an animal heart.
- Fig. 18: shows a scanning electron microscope image of a single curved portion of a filament of an endovascular prosthesis comprised of magnesium which has an applied surface protection layer of siloxane material. The prosthesis has been expanded to a diameter of 3.0mm and cracks in the protection layer are revealed
- Fig. 19: is a magnified image of a single crack in the protection layer which was previously applied to the surface of an endoprosthesis comprised of magnesium. The device has been expanded to a diameter of 3.0mm.
- Fig. 20: shows a scanning electron microscope image of an expanded endoprosthesis comprised of magnesium which has been coated with a siloxane material coated by plasma deposition using the monomer DVTMDSO.
- Fig. 21: shows a comparison of the radial strength of endoprostheses comprised of magnesium and with different coating configurations after undergoing bending and pulsatile repetitive cycling in a physiologically relevant environment for a period of twenty-eight days.

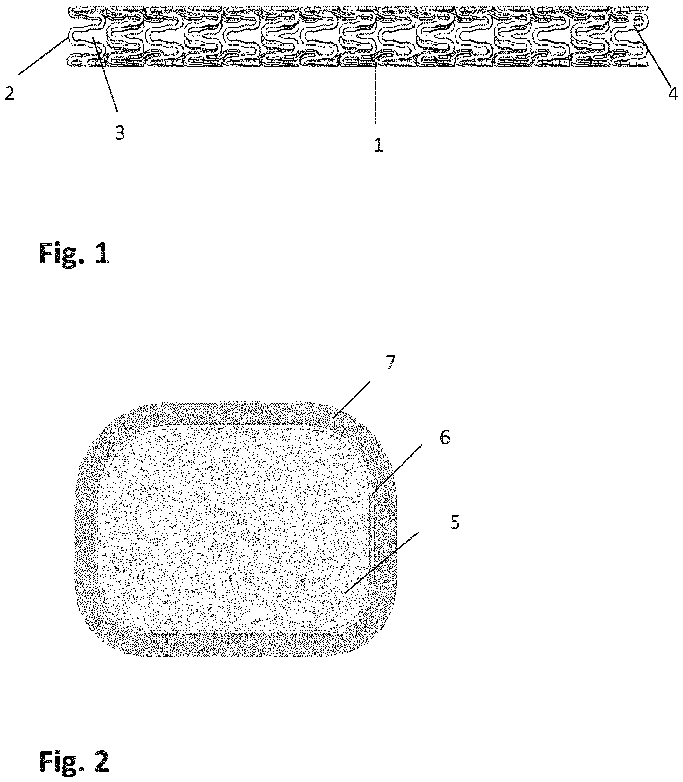

With reference to Fig. 1 an endoprosthesis (1) is illustrated which is generally tubular in nature and is comprised of inner diameter surfaces and outer diameter surfaces with additional surfaces created with the removal of material from a tube created from a base material. The device is comprised of undulating or serpentine rings (2) with open spaces between them (3) which are conjoined by linkages of the base material in a manner that the serpentine rings may be generally manipulated independent of one another. While the endoprosthesis is typically created by selective material removal means from a tube of base material it also possible to produce the device by welding serpentine wire rings, assembling from wire stock, or by forming it from a patterned piece of flat stock of material. The endoprosthesis is created from a bioabsorbable material which may not adequately contrast visually when subjected to fluoroscopic imaging, and therefore requires the addition of one or more radiopaque marker features (4).

After complete fabrication, the device will be compressed from its manufactured diameter to a lower diameter required for mounting on a percutaneous translumenal catheter angioplasty balloon. At a future time in clinical usage the catheter balloon will be expanded fully and the diameter of the stent will exceed its manufactured diameter to yet a larger deployed diameter. Those persons who are familiar with the design of endoprostheses will recognize that plastic deformation of particular features of the device will be required in order to facilitate the excursion of diameter from its initial to final state. Finite elemental analysis of such devices suggests that this plastic deformation may be in the range of 5 percent to 20 percent calculated as percentage strain by known engineering methods. An important consideration in placement of protective surface treatments or coatings is that any materials applied to and making direct contact with the outer surface would also undergo the plastic deformation at that surface or would fracture, and expose the underlying metallic base material.

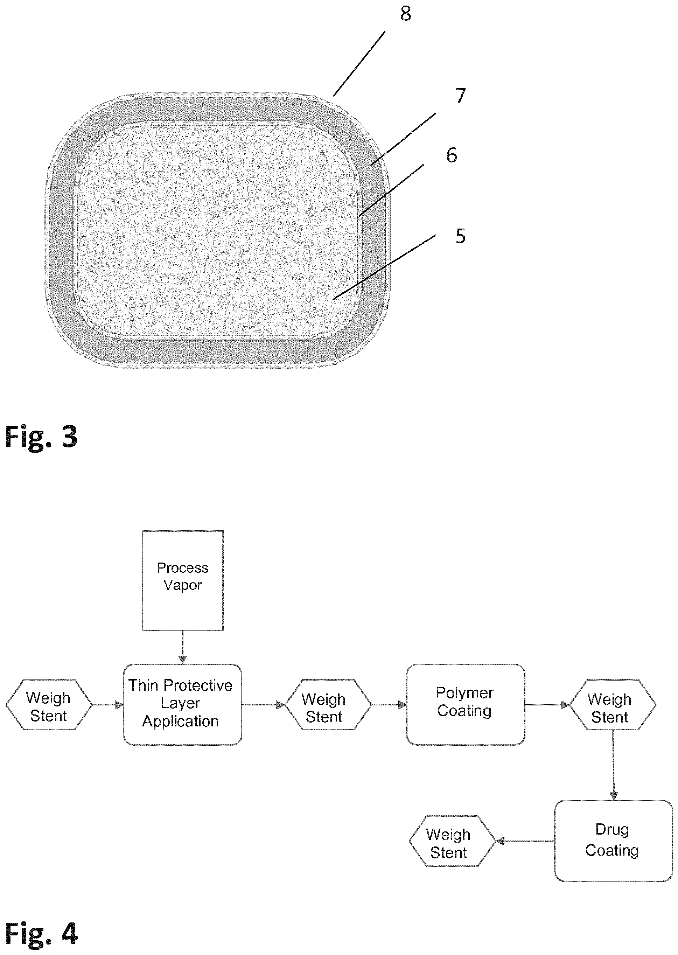

With reference to Figs. 2 and 3 cross-section illustrations of the endoprosthesis with multiple protective layers are presented. The base material (5) is comprised of a bioabsorbable metal. Examples of the base material(5) include but are not limited to of magnesium and alloys of magnesium, iron and iron alloys, aluminum and aluminum alloys, zinc and zinc alloys. One such material (5) is Elektron ZRE1 magnesium alloy which is obtained from Magnesium Elektron UK of Manchester, England. This material is comprised of magnesium with a 2.0%-3.0% zinc, 0.4%-1.0% zirconium, and 2.5%-4.0% of rare earth elements. The tensile strength of 160 MPa and yield strength of 110 MPa of this metal provide sufficient strength to create an endoprosthesis to support the radial support requirements of an endovascular device. Such materials may be further modified through metallurgical means to provide optimized corrosion resistance or tensile properties. Work hardening and heat tempering are exemplary means to manipulate or orient metallic grains in a preferential manner to increase fatigue life or tensile strength of a given bioabsorbable metal or metal alloy. The geometry of the device in the current invention is developed so that it will support physiological radial loads over a range of deployed diameters. Flexibility, bioabsorption rate, potential drug delivery characteristics, and physical size of the device in a vascular delivery system are also other design considerations in development of the device design.

Within Figs. 2 and 3 two protective layers (6, 7) are provided to prevent premature corrosion of the endoprosthesis. Corrosion protection of magnesium and aluminum is a well-established science. Dichromate treatments conforming to standard ASTM D1732 class 1 and galvanic anodize treatments conforming to standard ASTM D1732 class 2 are standard industrial treatments for magnesium under the trade names Dow #7 and Dow #9. The HAE process for magnesium provides good abrasion resistance and excellent corrosion resistance for coated devices as measured by hours of exposure to salt spray in industry standard testing. The Tagnite® process is an anodize process I which surface penetration and buildup of a dense magnesium oxide coating which covers the base metal surface to protect from environmental and potential galvanic corrosion. These traditional passivation treatments can be effective for application to static or nondeformable components that are generally not for implantable medical applications. Several requirements of implantable endoprosthesis devices preclude the use of traditional and well established magnesium passivation methods. First, any process material which is considered toxic, inflammatory, thrombogenic, or prevents tissue healing is not suitable for implant use. These materials would include chromates, nickel, and permanganate compounds. Second, an endoprosthesis is subject to plastic deformation and dynamic motion during manufacturing, deployment, and implant duration. Any coating or protective layer that would inhibit effective motion or deformation of the device through hardness or excessive thickness requirements would be prohibitive. Finally, in a device designed to be biodegradable or bioabsorbable the coating must be eventually be metabolized by the host animal along with the base material from which the device is constructed.

The protective layer (6) is considered a thin and dense passivation layer which provides primary protection of the base material (5) against corrosion by means of preventing fluid ingress from surrounding animal tissue. The protective layer is considered primarily a passivation means to prevent premature corrosion of the implantable device. In this regard this primary layer is nonporous and contiguous, covering the entire surface of the implant to provide complete corrosion protection. It is not used to deliver a drug or to enhance adhesion properties or promote the release of a drug by another coating layer or the base device itself. Thickness of this layer (6) can be measured by one or more methods used to measure thin layers commonly found in the electronics industry. Scanning electron microscope imaging of a sectioned device can reveal the dimensions of external coatings and layers. Spectroscopic means such as Auger elemental surface analysis can be utilized to determine layer depth. Interferometric or spectral reflectance means can also be used to determine the coating thickness of some transparent coatings. As an example, the model F40 layer thickness measurement tool from Filmetrics, Inc. (San Diego, CA) can be used to measure coating depths on areas as small as 1 micron diameter on the surface of a vascular implant. An effective thickness of layer (6) would range from about 20 nanometers to about 5 microns, and was found to be particularly effective from about 20 nanometers to about 1 micron.

The layer (6) may be deposited or may involve penetration or conversion of the base material to a more stable, yet bioerodable layer. It is required that the layer (6) provide adequate protection to the base metal for a temporary and predetermined time period before degradation. Those familiar with vascular implants will appreciate that a dense layer (6) should be only sufficiently thick enough to provide an effective temporary barrier and not more so. Increasing thickness of the layer (6) will increase the interface stresses which occur when the substrate undergoes bending movement which will increase the potential for delamination or cracking of this layer and subsequent exposure of the base material(5).

A layer (6) which is too thick may also impart undesired rigidity to the endoprosthesis (1) and then may diminish the ability to deliver and deploy the device in clinical usage.

A suitable protective layer (6), not according to the invention, can be created on the base metal (5) surface of the endoprosthesis by exposing it to heat in an oxygen environment. When magnesium is the base metal a heat treatment in the range of 400°C to 440°C will be sufficient to create a surface oxide when the device is exposed to heat for a time period in the range of 3 to 10 minutes. With the intent of treating only the metallic surface care is taken not to expose the device to excessive high temperature or heat duration that would alter the grain structure or metallurgy to affect the tensile or fatigue properties of the device.
A thicker surface oxide may be created on the endoprosthesis by first etching the surface with a sodium bicarbonate solution and then applying heat treatment. First, a 0.5 molar solution of sodium bicarbonate NaHCO₃ in deionized water is prepared. Next, the untreated metal device is immersed in this solution for three to five minutes at room temperature. After rinsing with deionized water and complete drying in air the device is subject to heat treatment in the range of 400°C to 440°C which will be sufficient to create a surface oxide with increased thickness when the device is exposed to heat for a time period in the range of 3 to 10 minutes.
Alternatively, a suitable protective layer can be created on the base metal (5) surface of the endoprosthesis by plasma assisted deposition of siloxane material. The plasma assisted deposition is realized by using an injected suitable monomer compound or compounds. At least one suitable monomer compound can be selected from the group comprising or consisting of hexamethyldisiloxane, divinyltetramethyldisiloxane, methyltrimethoxysilane, tetramethylsiloxane, hexamethyldisiloxane, tetramethylsilane, tetramethoxysilane. In a preferred example a suitable protective layer can be created on the base metal (5) surface of the endoprosthesis by plasma assisted deposition of using an injected organosilicon compound as suggested herein such as hexamethyldisiloxane (HMDSO) liquid. In this process, an atmospheric pressure plasma discharge in argon gas with small admixtures of the organosilicon compound, e.g. HMDSO, is employed for the deposition of thin layers comprised of chained and unchained dimethylsiloxane molecules to form a siloxane barrier. The resulting layer is an effective moisture barrier with good adhesion to the base metal substrate and flexibility. This process further benefits from a low temperature environment. Unlike spray coating of individual devices, many devices can be coated simultaneously with very uniform deposition depths. Equipment for deposition of a siloxane layer can be obtained from Plasma Technology Systems of Belmont, CA or from Materion GmbH of Wismar, Germany. Similarly, the plasma process can be used to deposit glass-like layers similar to silicon dioxide. However, the ductility of silicon dioxide or SiOₓ layers may be more rigid and may produce more cracking which is evident when the device is compressed onto a delivery balloon and expanded to a clinically appropriate diameter. The thin layer protective layer (6) can also be created from plasma deposition of hydrocarbon materials including polypropylene or polyethylene when the appropriate precursor materials are applied into the plasma deposition chamber.

Suitable gentle process parameters for creating a protective layer on the base metal (5) surface of the endoprosthesis by plasma assisted deposition of organic and/or siloxane material are listed below. In terms of RF frequency a range of 10KHz to 2.5 GHz is applicable. In the plasma chamber pressure can be applied ranging from 0.01 torr to 2 torr. Only relatively low power is necessary for deposition ranging from 10 watts to 700 watts. Moreover, low temperatures in the range from about 23 °C (about room temperature) to about 60 °C can be applied to deposit the organic protective layer. Those temperatures are suitable to prevent both the base material and the deployed organic material from damage. Generally the carrier gas is selected from Argon, Nitrogen, Oxygen and CO₂ at gas flow rates from 0.17 sccm to 42 sccm.

Upon compression and expansion of the device while being used certain features of the device geometry may be subject to plastic deformation of approximately 15% of the original feature dimension. The dense, thin protective layer (6) which covers the device must then deform by an equal value which could lead to cracking and subsequent exposure of the base material (5). In clinical usage the deployed device may be subject to bending movement, pulsatile motion, stretching, and torsion depending on the anatomical location of deployment. These physiological motions may further exacerbate any crack or fissure which may be present. Cracking and subsequent exposure of the base material (5) may occur to a higher extent if the thin protective layer (6) does not exhibit sufficient elasticity.

It is therefore beneficial to place according to the invention a biodegradable polymer layer (7) which will further protect the device and create a flexible seal over the thin protective layer (6) in the event of cracks which may occur during deployment and clinical usage. Representative examples of polymers that may be used to create polymer layer (7) to coat a bioabsorbable endoprosthesis include, but are not limited to poly(caprolactone), polyvinyl esters, poly(D,L lactic acid), poly(L-lactide), poly(lactid-co-glycolide), polyorthoester, polyanhydride, poly(trimethylene carbonate), poyurethanes, silicones, oils, and fatty acids. The polymer layer (7) should be biocompatible and not induce inflammation when implanted into an animal body. The polymer should be soluble in standard solvents which may be later evaporated from the device by vacuum drying in a heated environment. The polymer should have a glass transition point T_{g} which is higher than any process used to fabricate the finished device, including the sterilization process. The polymer layer (7) should have suitable flexibility to maintain its consistency and not produce cracks upon deployment of the device or during clinical usage of the device. It should also have sufficient mechanical strength to maintain its shape and not deform during manufacturing processes, deployment, or clinical life of the device. Finally, the polymer must be biodegradable within the predetermined clinical usage life of the device.

An exemplary polymer layer (7) is formed of PC-12 polycaprolactone which has a tensile modulus of elasticity in the range of 0.2-0.3GPa, a tensile strength of 25-30 MPa, a value for elongation to break of greater than 300%, and a degradation time of 12 - 24 months. In an alternative embodiment of the invention a blend of polymers may be used. Blending a flexible polymer such as polycaprolactone with a more rigid polymer such as poly L-lactide may yield a better combination of properties which include mechanical strength, flexibility, elongation at break, or degradation time which are relevant to the particular application of the device. Further, it is possible to blend or to copolymerize biodegradable polymers to optimize the beneficial properties required.

A therapeutic drug may be added to polymer layer (7) or an additional drug or drug and polymer combination layer (8) may be applied to polymer layer (7). It is known that a therapeutic drug may be beneficial to reduce the incidence of restenosis of a vascular lesion in which an endovascular prosthesis has been placed. Appropriate drugs include but are not limited to those with antiproliferative, immunosuppressive, antineoplastic, antithrombogenic, or other clinically beneficial properties. Exemplary anti-platelet, fibrinolytic, or thrombolytic agents are heparin, aspirin, hirudin, ticlopidine, urokinase, and mixtures thereof. Macrocyclic triene molecules of the rapamycin family are commonly used in drug eluting stent applications. This class of drugs includes alone or in combination with a biodegradable polymer coating those selected from a group which includes the compounds Rapamycin, Biolimus A9, Everolimus, Zotarolimus, CRC-015, Novolimus, Tacrolimus, and Myolimus and combinations thereof. Anticancer drugs for drug eluting endoprosthesis use include taxol, paclitaxol, doxorubicin, antisense compounds and combinations thereof.

The drug may be incorporated into polymer layer (7) as a drug matrix. After implantation, the drug diffuses out of the polymer matrix and preferably into the surrounding tissue over a period lasting at least 4 weeks, and in some cases up to 3 months or longer, ideally matching the time course of restenosis, smooth muscle cell proliferation, thrombosis, or a combination thereof. In addition to diffusion, the drug may also be released as the polymer degrades or dissolves, making the drug more readily available to the surrounding tissue. The ratio of drug to polymer used in the matrix can range from preferably 0 percent to 70 percent by weight depending on the potency and mode of action of the drug and the physical character of the polymer material which is used. If a drug is contained in polymer layer (7) then any additives may affect the properties of the polymer and increasing the drug to polymer ration may impart a reduction in the degradation time or structural integrity of the material and its ability to adequately cover and seal the thin protective layer (6) and base material(5). Some embodiments of the invention would have an additional drug or drug and polymer combination (8). In this configuration the drug or drug and polymer combination (8) is completely independent of polymer layer (7) and the drug release kinetics would not affect the properties of polymer layer (7) below. Representative examples of polymers that may be used to create a drug and polymer layer(8) include, but are not limited to poly(caprolactone), polyvinyl esters, poly(D,L lactic acid), poly(L-lactide), poly(lactid-co-glycolide), polyorthoester, polyanhydride, poly(trimethylene carbonate), poyurethanes, silicones, oils, and fatty acids. The thickness of polymer layer (7) is preferably from 1 micron to 30 microns. The thickness of the drug or drug and polymer combination (8) is preferably from 1 to 10 microns. It should not be necessary to add an additional diffusion-controlling layer to the polymer layer (7) or polymer layer (8).

A sequence of fabrication steps is required to create the invention. The schematic representation of Fig 4 illustrates the sequence of steps required to create one embodiment of the invention. A weight measurement step is included between each process to assist in evaluation of the mass generated by each step.

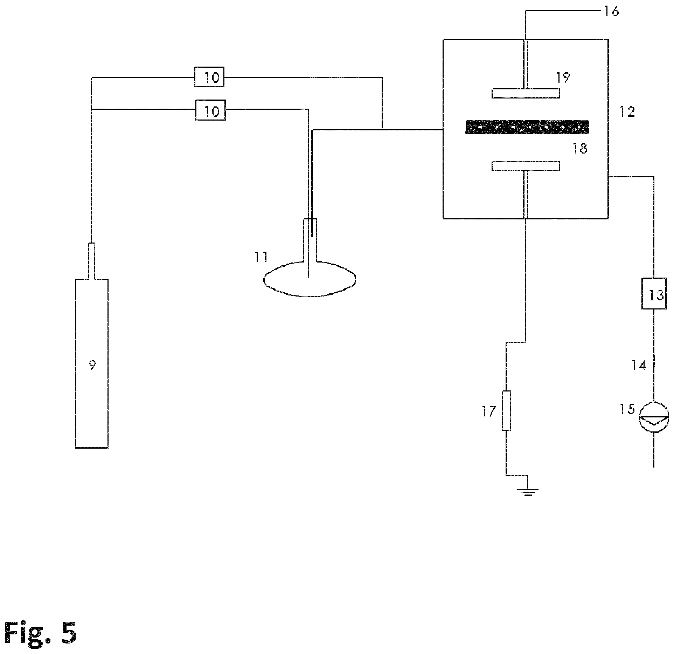

Fig. 5 illustrates a schematic diagram of typical equipment used to deposit a thin protective layer (6) in an embodiment where the layer is created from monomer units as suggested herein, for example of liquid monomer solution of hexamethyldisiloxane (HMDSO), injected into a plasma chamber. An inert gas (9) such as nitrogen or argon is used as a carrier gas and to create a gas vapor from a reservoir of the exemplary chosen liquid HMDSO (11), while the other herein suggested monomer compounds will work all the like. Mass flow controllers (10) are used to regulate the gas flow. The combined vapor and carrier gas are introduced into a plasma enclosure (12). Within this enclosure (12) one or more endoprosthesis (18) is placed between two electrodes (19). One electrode is connected to an alternating power source (16) and the other electrode is connected to ground through resistor (17). A pressure gauge (13) measures internal chamber pressure which can be regulated by adjustment of gas through the mass flow controllers (10) and the vacuum pump (15) across valve (14). Power is applied across the electrodes to generate a plasma at near atmospheric pressure with subsequent deposition of for example dimethylsiloxane molecules and molecular radicals and combinations of these molecules. The thickness of the layer deposition is dependent on the applied power, the monomer concentration within the carrier gas, and the rate of gas flow among other factors. For example, a siloxane layer with a thickness of 200 nM to 400 nM will have a typical tensile strength of 25 MPa and a resulting value of 130 to 150 strain% at break. Siloxane layers are known to those familiar with medical device coatings to be effective moisture barriers with limited ductility.

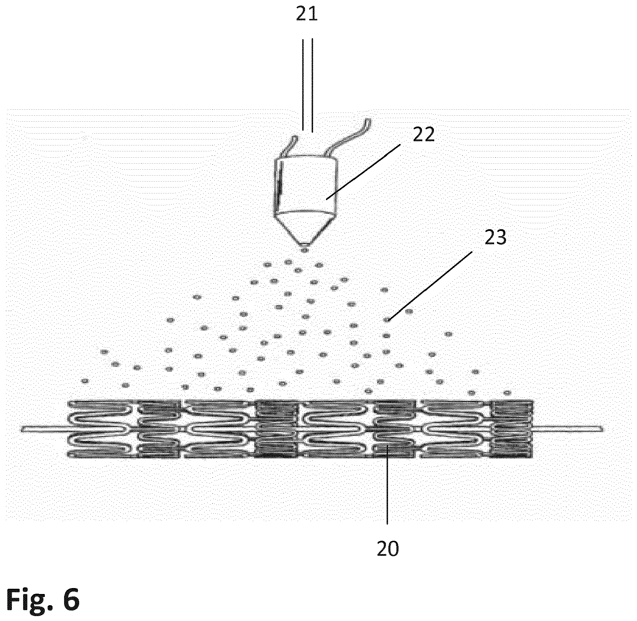

The thin protective layer (6) alone may provide effective corrosion protection to the base material (5) of the endoprosthesis. In a preferred embodiment an additional polymer layer (7) may be applied which partially or fully covers the protective layer (6). An apparatus to apply the polymer layer (7) is illustrated in Fig. 6. The coating formulation can be applied by spray coating, dip coating, casting, vapor deposition, manual application with a syringe, or other means. In a preferred embodiment a 120kHz Micromist nozzle(22) from Sono-tek Inc., (Milton, New York), is used to deposit a coating of between 10 and 35 microns depth of polymer coating onto all surfaces of the device. A solution of polymer and solvent (21) is applied to an appropriate spray nozzle (22) by pressure means. The concentration of polymer in solvent solution may range from 10 mg/ml to 100 mg/ml. which may be adjusted to obtain an optimum droplet size. Compressed air or an inert gas may be used to direct the spray droplets or to segregate droplets within the spray stream. The solvent chosen for the formulation should preferably dissolve the polymer completely to create a clear solution without the appearance of undissolved matter. Solvents with high vapor pressures permit ease of removal from the sprayed polymer when placed in a heated vacuum chamber such as Model 1410M from VWR International (Sheldon Manufacturing, Cornelius, OR) set to a temperature between 25 °C and 55 °C and an applied vacuum which ranges from 15 inHg to 29 inHg for a time period of between 1 hour and 72 hours.

Solvents should be chosen with preference to those which do not exhibit a toxic or inflammatory response in vascular tissue. Examples of solvents which may be considered for use in embodiments of this invention include but are not limited to acetone, chloroform, ethyl lactate, dichloromethane, methylene chloride and combinations thereof. The droplets of polymer in solvent (23) are uniformly dispensed on all of the surfaces of a prosthesis device which has been positioned and subsequently rotated or translated to the correct position to accept the droplets to create a uniform surface coating. A therapeutic drug may be added to the solution of polymer and solvent (21).

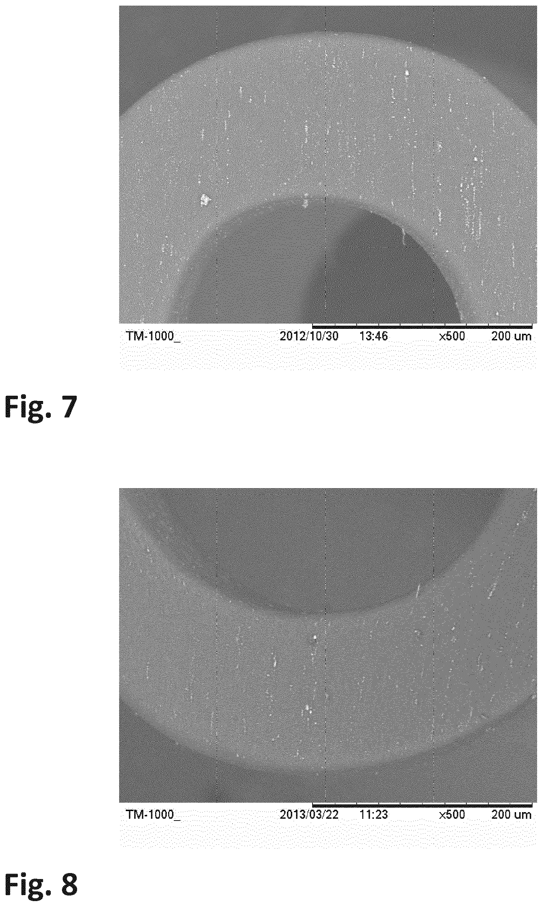

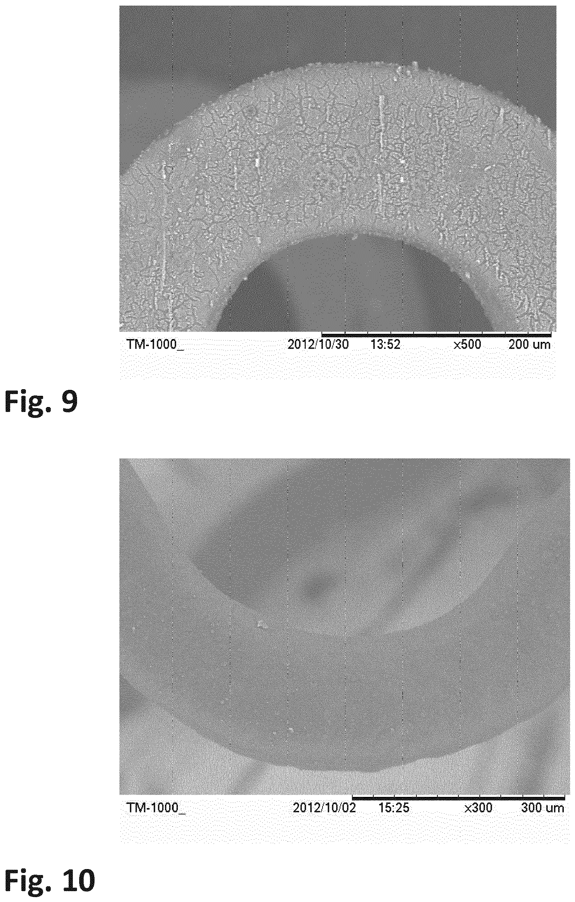

Figs. 7 through 10 are scanning electron microscope images of a preferred embodiment of the invention during various stages of assembly. Fig. 7 is an image of a feature of an endoprosthesis fabricated from a magnesium alloy. Fig. 8 is an image of a feature of an endoprosthesis fabricated from a magnesium alloy which has been coated with a thin protective layer of dimethylsiloxane molecules which is approximately 250 nM in thickness. Fig. 9 is an image of a feature of an endoprosthesis fabricated from a magnesium alloy which also incorporates a thin protective layer. This layer was created by immersing the device in a sodium bicarbonate solution at room temperature for a period of five minutes followed by rinsing in deionized water and drying to complete dryness. The device was then heated in an oven for five minutes at 426°C to produce a stable oxide layer over the surfaces of the device. Fig. 10 is an image of a feature of an endoprosthesis fabricated from a magnesium alloy which contains multiple protective layers which include in this example a thin protective layer (6) a polymer layer (7) and a drug-containing polymer layer (8).

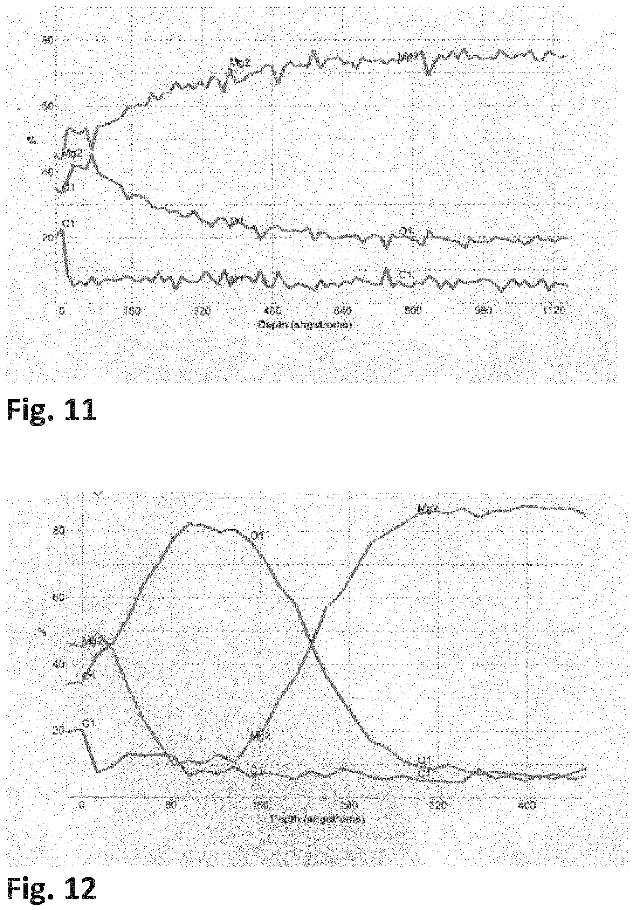

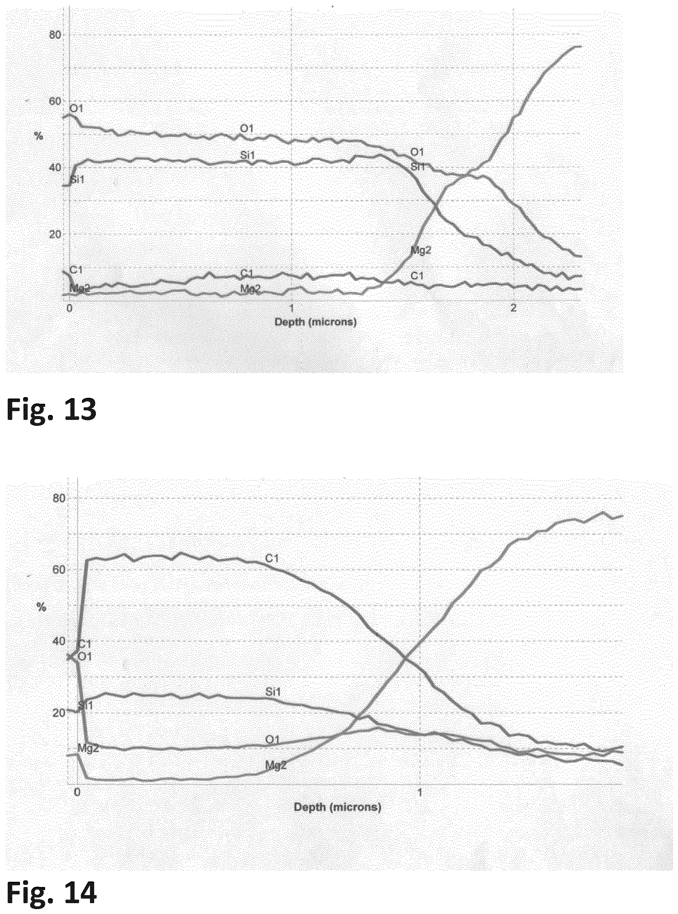

Figs. 11 through 14 show surface elemental analysis scans of the surface of a magnesium alloy stent and stents which have been processed sequentially in accordance with a preferred embodiment of the invention. The surface scans were performed with a PHI model 600 spectroscope from Physical Electronics, Inc. (Chanhassen, MN) with an applied voltage of 3 kV. Fig. 11 shows the surface scan of an untreated endoprosthesis fabricated from a magnesium alloy. The predominant element on the device surface is magnesium. Fig. 12 shows the surface scan of an endoprosthesis fabricated from a magnesium alloy which has been placed into a heated chamber at 426°C for a period of five minutes. An oxide layer of approximately 200 Angstroms thickness is evident from the surface scan as noted by the presence of elemental oxygen that is not apparent in the surface scan of the untreated magnesium alloy stent. Fig. 13 shows the scan of the surface of an endoprosthesis fabricated from a magnesium alloy which has been placed into a plasma chamber and subjected to active gas plasma in the presence of silicon and oxygen molecules for a period of fifteen minutes. A glass-like layer of SiOₓ of approximately 1 to 2 microns thickness is evident from the surface scan as noted by the presence of silicon and oxygen that is not apparent in the surface scan of the untreated magnesium stent. Fig. 14 shows the surface of an endoprosthesis fabricated from a magnesium alloy which has been placed into a plasma chamber and subjected to active gas plasma in the presence of silicon, carbon, and oxygen atoms for a period of fifteen minutes. A layer of siloxane molecules of approximately 500 nM to 800 nM in thickness is evident from the surface scan as noted by the presence of carbon, silicon, and oxygen that is not apparent in the surface scan of the untreated magnesium stent.

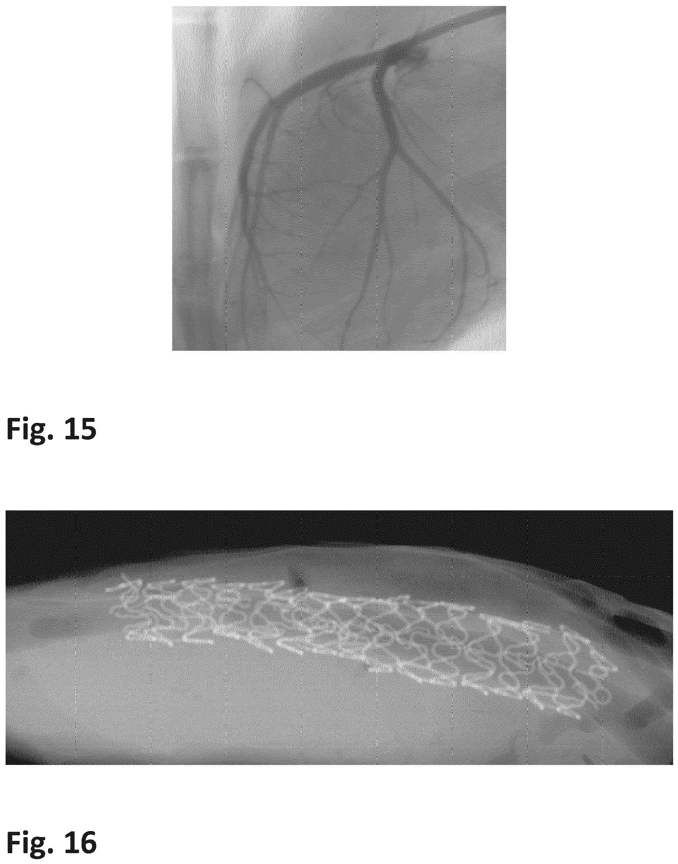

Fig. 15 shows an angiogram of the left anterior descending coronary artery of a living porcine heart in which is deployed an endoprosthesis which has been processed in accordance with methods described in the present invention. The implanted device was fabricated by first coating a stent made from a magnesium alloy with a protective layer of polycaprolactone polymer using materials and methods described herein. It was then coated with a drug and polymer coating layer comprised of the rapamycin derivative drug CRC-015 combined with the polymer d,l PLA. After removal of all solvents in a heated vacuum chamber the device was mounted on a delivery balloon and packaged in protective foil packaging and then sterilized with ethylene oxide gas.

The device was deployed into the animal heart and the angiogram was produced twenty-eight days after the initial procedure. At this later time point the endoprosthesis provided adequate vessel patency as indicated by the contrast image of the vessel cross-section of the proximal portion of the left anterior descending coronary artery of the animal heart.

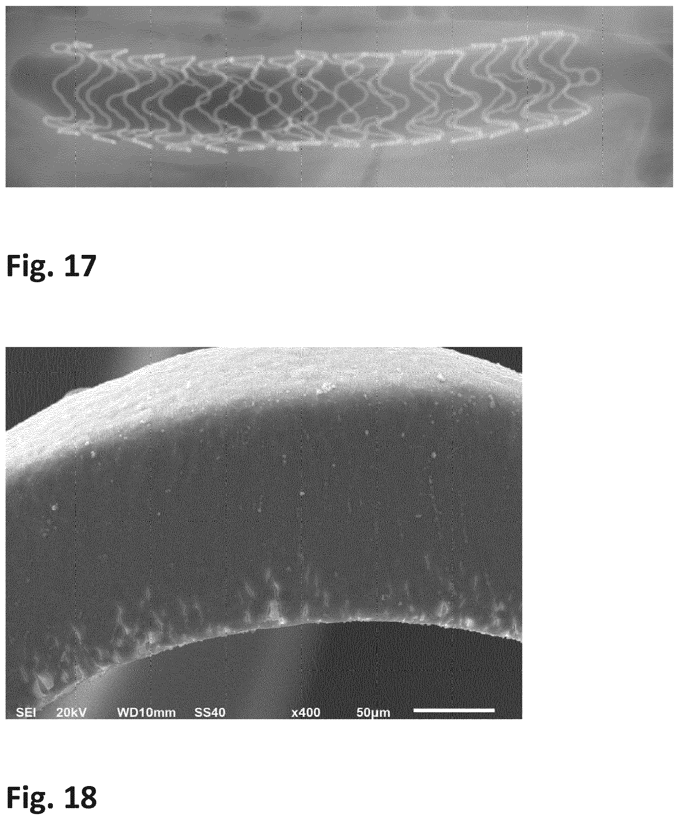

A biodegradable endoprosthesis which does not maintain adequate support for the period of time required for vessel healing may fracture and exhibit compression and loss of diameter. The vessel lumen may be reduced as a direct consequence of the stent compression Fig. 16 shows an X-ray image of an implanted endoprosthesis comprised of magnesium with other materials and processed in a manner that does not provide adequate protection to the base material which is provided twenty-eight days after the endoprosthesis has been deployed into a coronary artery of an animal heart. In contrast, a biodegradable endoprosthesis which does maintain adequate support for the period of time required for complete vessel healing will maintain its diameter and should begin to fracture after completed healing of the vessel. Fig. 17 shows an X-ray image of an endoprosthesis comprised of magnesium and processed in a manner consistent with methods and processes specified herein to provide adequate protection to the base material which is provided twenty-eight days after the endoprosthesis has been deployed into a coronary artery of an animal heart. The ability of a device to support loads imparted to it by surrounding vessel tissue is an indication of its scaffolding characteristic. Loading may be radial, bending, axial compression, axial tension, torsion, or a combination these forces. Fractures of the features of a biodegradable endoprosthesis due to localized corrosion will reduce the scaffolding capability as will ductile deformation of the device. The explanted device seen in the X-ray image of Fig. 17 appears to be free of separation fractures and has closely maintained its original diameter at time of implant.

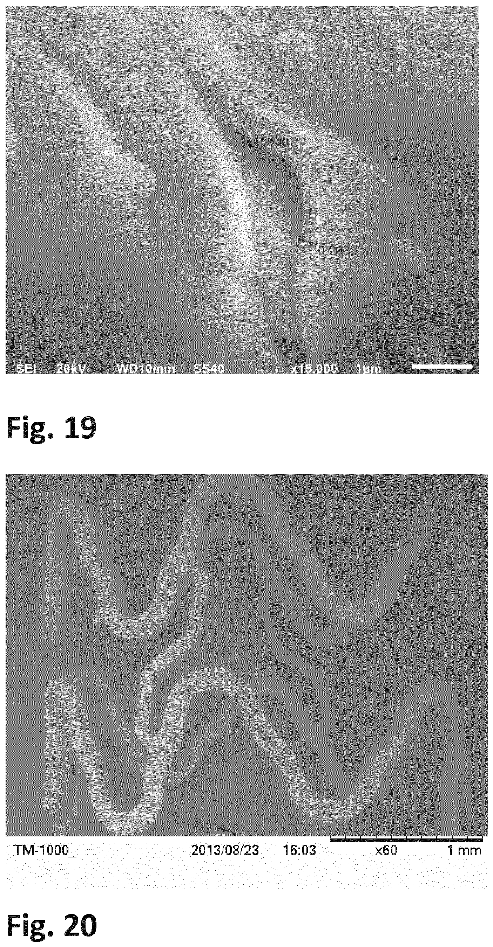

If the endoprosthesis has no corrosion protection or inadequate applied corrosion protection then fluid electrolytes from the vascular tissue which surrounds the implant will permeate the base metal of the device and oxidation of the base metal will occur. If a thin protective layer is applied and a crack or fissure develops in the layer due to expansion of the device or material fatigue from physiological motions then fluid electrolyte ingress to the base metal will occur. The corrosion process will initiate even if the cracks in the thin protective layer are very small, but the process will be retarded in comparison to no protection at all. Fig 18 is a scanning electron microscope image of a biodegradable endoprosthesis with a base metal formed of a magnesium alloy. The original diameter of the device at the time of fabrication and coating with a thin layer of siloxane from vaporized HMDSO monomer is approximately 2.0 mm. When the device is then mounted onto a delivery catheter balloon it will assume a new diameter of approximately 1.3 mm. Finally, in a clinical application the device will be expanded by the balloon to a final diameter of approximately 3.0 mm. At the largest expanded diameter very minute fissures may be seen in the features of the device under high magnification as seen in Fig. 18 and Fig. 19. These fissures may typically range in width from 0.1 microns to 1.0 microns and in length from 1 micron to 50 microns. The thickness of the applied layer is an important consideration if the substrate is to be subjected to bending. In general applications a higher coating thickness will yield a higher density of fissures. When applied to the surface of a biodegradable endoprosthesis the thickness of the thin protective layer is in the approximate range of 20 nanometers to about 5 microns, preferably in the range of 50 nanometers to about 3 micron, more preferably in the range of 75 nanometers to 1 micron, and most preferably in the range of 200 nanometers to 600 nanometers to yield minimum crack or fissure creation. Thicker coatings may yield a greater density of fissures and are therefore less preferred. A thinner layer coating in the range of 0 to 20 nM may not withstand the deleterious effect of animal body fluid electrolytes, enzymes, or macrophage action. Altering the liquid monomer material may provide a beneficial effect in reducing cracking. For instance, in Fig. 20 the liquid monomer used to create the siloxane coating is changed to DVTMDSO, a vinyl-containing chemical which upon plasma deposition yields a more flexible coating. In this case there is no cracking which is evident upon expansion of the stent.

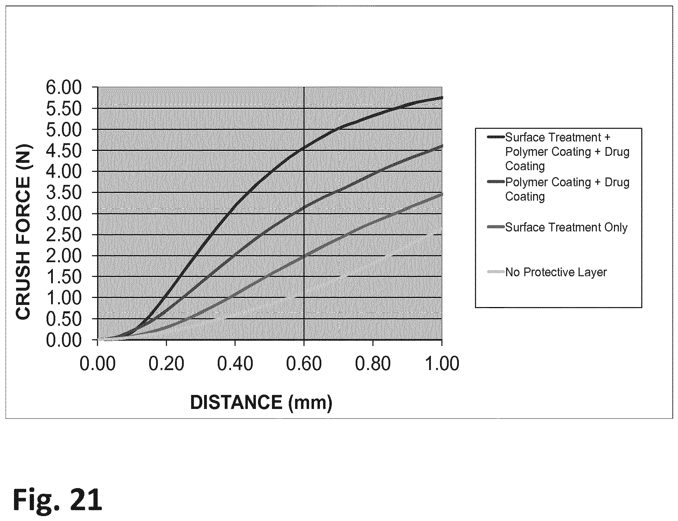

Due to excessive strain at the time of deployment and fatigue which results from physiological repetitive movement undesirable fissures may form at a premature time, initiating the process of base metal corrosion. It may therefore be highly beneficial to also apply a layer of biodegradable polymer coating that may be thicker than the thin protectivelayer and may be more elastic in order to protect the thin protective layer and to effectively prevent or seal undesirable microcracks and fissures which may develop in the thin protective layer over time. Fig. 21 is a graphical illustration of the results of testing four groups of biodegradable endoprostheses comprised of a magnesium alloy. Devices in three groups were coated with protective thin layers. In one group a thin protective layer comprised of approximately 250 nM in coating thickness of siloxane was applied. And in yet another group both the thin protective layer of siloxane and the thicker layer of polycaprolactone were applied. The group with the polycaprolactone polymer layers also received a thin drug eluting coating which consisted of 160 micrograms of polylactide polymer and 160 micrograms of rapamycin drug. All sample sets were expanded to 3.0 mm into silicone tubes with dimensions and physical attributes which would correlate to coronary arteries. The tubes were filled with porcine serum through a heated pumping circuit. The silicone tubes were bent at an angle and movement cycle corresponding to a human left anterior descending artery. The implants were visually observed at regular intervals and their appearance was recorded digitally. The stent samples were maintained cycling in the test apparatus for thirty days, a period of time representing one month of life in a human coronary artery. At the end of this time period the tubes were removed from the apparatus and flushed with deionized water. The vessels with the stents still inside were placed into a test jig and crushed with an Imada model 25 compression tester with a load cell rated at 25 Newtons compressive force. Those stents which were not treated were visually broken and did not retain sufficient residual radial force. The group of stents which were treated with the thin protective siloxane layer maintained a radial force of approximately 2.0 N required to compress the deployed stent to approximately 80% of their deployed diameter. The group of stents which were treated with the thin protectivesiloxane layer and the biodegradable polymer maintained a radial force of approximately 4.5 N required to compress the deployed stent to approximately 80% of their deployed diameter.

## Claims

1. An expandable prosthesis for vascular placement comprising, a tubular biodegradable metal structure, a biodegradable organic layer covering the surfaces of the structure, and a biodegradable polymer coating covering the organic surface layer, wherein the biodegradable organic layer comprises an organic compound applied by injection of monomer vapor into a chamber with an applied plasma and wherein thickness of the biodegradable organic layer ranges from 20 nanometers to 5 micrometers (microns), wherein the biodegradable organic layer is a siloxane layer.

2. The expandable prosthesis of claim 1, wherein the optional biodegradable polymer coating contains a therapeutic drug.

3. The expandable prosthesis of claim 1 or 2, wherein the biodegradable polymer coating contains a therapeutic drug selected from a group which includes the compounds Rapamycin, Biolimus A9, Everolimus, Zotarolimus, CRC-015, Novolimus, Tacrolimus, and Myolimus.

4. The expandable prosthesis according to any of the preceding claims, wherein the optional polymer coating contains a therapeutic drug selected from the group which includes paclitaxel and paclitaxel derivative compounds.

5. The expandable prosthesis according to any of the preceding claims, wherein the monomer vapor is preferably created from at least one of the following compounds hexamethyldisiloxane, divinyltetramethyldisiloxane, methyltrimethoxysilane, tetramethylsiloxane, hexamethyldisiloxane, tetramethylsilane and tetramethoxysilane.

6. The expandable prosthesis of claim 5, wherein the biodegradable organic layer is a uniform polymethylsiloxane layer applied by injection of HMDSO (hexamethyldisiloxane) vapor into a chamber with an applied plasma.

7. The expandable prosthesis of claim 5, wherein the biodegradable organic layer is a uniform polymethylsiloxane layer applied by injection of DVTMDSO (divinyltetramethyldisiloxane) vapor into a chamber with an applied plasma.

8. The expandable prosthesis according to any of the preceding claims, wherein the biodegradable metal is magnesium or a metallic alloy of magnesium.

9. The expandable prosthesis according to any of the preceding claims, wherein the optional polymer coating is selected from a group which includes the compounds cellulose, collagen, albumen, casein, polysaccharides, polylactide (PLA), poly-L-lactide(PLLA), poly-d,l-Lactide (PDLLA), polyglycol (PGA), polycaprolactone (PCL), poly-d,l-lactide-co-glycolide (PDLLA-PGA), polyhydroxybuteric acid (PHB), polyhydroxyvaleric acid (PHV), polyalkyl carbonates, polyorthoesters, polyethylene terepthalate (PET), polymalonic acid (PML), polyanhydrides, polyphosphazenes, polyaminoacids and their copolymers, hylauronic acid, and blends of the aforementioned compounds.

10. The expandable prosthesis according to any of the preceding claims, wherein the prosthesis is a stent and the therapeutic drug is contained on the surface of the biodegradable polymer layer.

11. The expandable prosthesis according to any of the preceding claims, wherein the prosthesis is a stent and the therapeutic drug is dispersed in the biodegradable polymer layer in a drug-to-polymer ratio of 10 percent by weight to 70 percent by weight.

12. The expandable prosthesis according to any of the preceding claims, wherein the prosthesis is a stent and the therapeutic drug is dispersed in the drug-containing polymer layer in a drug-to-polymer ratio of 10 percent by weight to 70 percent by weight.

13. A method of producing a biodegradable stent comprising
coating a tubular biodegradable metal structure with a biodegradable organic layer, and
coating the organic layer with a biodegradable polymer coating, wherein the biodegradable organic layer is a siloxane layer.

14. The method of claim 13, wherein the biodegradable organic layer is coated with a polymer coating containing a therapeutic drug.

15. The method of claim 13 or 14, wherein coating the tubular biodegradable metal structure with a biodegradable organic layer comprises coating the metal structure with a siloxane compound by a plasma enhanced chemical vapor deposition process.

## Patentansprüche

1. Aufweitbare Prothese zur Einpflanzung in ein Gefäß, die eine röhrenförmige biologisch abbaubare Metallstruktur, eine biologisch abbaubare organische Schicht, die die Oberflächen der Struktur bedeckt, und eine biologisch abbaubare Polymerbeschichtung umfasst, die die organische Oberflächenschicht bedeckt, wobei die biologisch abbaubare organische Schicht eine organische Verbindung umfasst, die durch Einspeisen von Monomerdampf in eine Kammer mit einem erzeugten Plasma aufgebracht wird, und wobei die Dicke der biologisch abbaubaren organischen Schicht von 20 Nanometer bis 5 Mikrometern (Mikron) reicht, wobei die biologisch abbaubare organische Schicht eine Siloxanschicht ist.

2. Aufweitbare Prothese nach Anspruch 1, wobei die optionale biologisch abbaubare Polymerbeschichtung ein therapeutisches Medikament enthält.

3. Aufweitbare Prothese nach Anspruch 1 oder 2, wobei die biologisch abbaubare Polymerbeschichtung ein therapeutisches Medikament enthält, das aus einer Gruppe ausgewählt ist, die die Verbindungen Rapamycin, Biolimus A9, Everolimus, Zotarolimus, CRC-015, Novolimus, Tacrolimus und Myolimus umfasst.

4. Aufweitbare Prothese nach einem der vorhergehenden Ansprüche, wobei die optionale Polymerbeschichtung ein therapeutisches Medikament enthält, das aus der Gruppe ausgewählt ist, die Paclitaxel und Paclitaxelderivat-Verbindungen umfasst.

5. Aufweitbare Prothese nach einem der vorhergehenden Ansprüche, wobei der Monomerdampf vorzugsweise aus mindestens einer der folgenden Verbindungen Hexamethyldisiloxan, Divinyltetramethyldisiloxan, Methyltrimethoxysilan, Tetramethylsiloxan, Hexamethyldisiloxan, Tetramethylsilan und Tetramethoxysilan erzeugt ist.

6. Aufweitbare Prothese nach Anspruch 5, wobei die biologisch abbaubare organische Schicht eine gleichmäßige Polymethylsiloxan-Schicht ist, die durch Einspeisen von HMDSO-Dampf (Hexamethyldisiloxan-Dampf) in eine Kammer mit einem erzeugten Plasma aufgebracht wird.

7. Aufweitbare Prothese nach Anspruch 5, wobei die biologisch abbaubare organische Schicht eine gleichmäßige Polymethylsiloxan-Schicht ist, die durch Einspeisen von DVTMDSO-Dampf (Divinyltetramethyldisiloxan-Dampf) in eine Kammer mit einem erzeugten Plasma aufgebracht wird.

8. Aufweitbare Prothese nach einem der vorhergehenden Ansprüche, wobei das biologisch abbaubare Metall Magnesium oder eine Magnesium-Metalllegierung ist.

9. Aufweitbare Prothese nach einem der vorhergehenden Ansprüche, wobei die optionale Polymerbeschichtung aus einer Gruppe ausgewählt ist, die die Verbindungen Cellulose, Kollagen, Albumen, Kasein, Polysaccharide, Polylactid (PLA), Poly-L-Lactid (PLLA), Poly-D,L-Lactid (PDLLA), Polyglykol (PGA), Polycaprolacton (PCL), Poly-D,L-lactid-co-Glycolid (PDLLA-PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und ihre Copolymere, Hylauronsäure und Mischungen aus den vorgenannten Verbindungen umfasst.

10. Aufweitbare Prothese nach einem der vorhergehenden Ansprüche, wobei die Prothese ein Stent ist und das therapeutische Medikament auf der Oberfläche der biologisch abbaubaren Polymerschicht enthalten ist.

11. Aufweitbare Prothese nach einem der vorhergehenden Ansprüche, wobei die Prothese ein Stent ist und das therapeutische Medikament in der biologisch abbaubaren Polymerschicht in einem Medikament-zu-Polymer-Verhältnis von 10 Gewichtsprozent bis 70 Gewichtsprozent verteilt ist.

12. Aufweitbare Prothese nach einem der vorhergehenden Ansprüche, wobei die Prothese ein Stent ist und das therapeutische Medikament in der medikamenthaltigen Polymerschicht in einem Medikament-zu-Polymer-Verhältnis von 10 Gewichtsprozent bis 70 Gewichtsprozent verteilt ist.

13. Verfahren zum Herstellen eines biologisch abbaubaren Stents, umfassend Beschichten einer röhrenförmigen biologisch abbaubaren Metallstruktur mit einer biologisch abbaubaren organischen Schicht, und
Beschichten der organischen Schicht mit einer biologisch abbaubaren Polymerbeschichtung, wobei die biologisch abbaubare organische Schicht eine Siloxanschicht ist.

14. Verfahren nach Anspruch 13, wobei die biologisch abbaubare organische Schicht mit einer Polymerbeschichtung beschichtet ist, die ein therapeutisches Medikament enthält.

15. Verfahren nach Anspruch 13 oder 14, wobei das Beschichten der röhrenförmigen Metallstruktur mit einer biologisch abbaubaren organischen Schicht ein Beschichten der Metallstruktur mit einer Siloxanverbindung über ein plasmaunterstütztes chemisches Gasphasenabscheidungsverfahren umfasst.

## Revendications

1. Prothèse expansible destinée à une mise en place vasculaire comprenant une structure métallique biodégradable tubulaire, une couche organique biodégradable recouvrant les surfaces de la structure, et un revêtement de polymère biodégradable recouvrant la couche de surface organique, la couche organique biodégradable comprenant un composé organique appliqué par injection d'une vapeur de monomère dans une chambre avec un plasma appliqué et l'épaisseur de la couche organique biodégradable allant de 20 nanomètres à 5 micromètres (microns), la couche organique biodégradable étant une couche de siloxane.

2. Prothèse expansible selon la revendication 1, dans laquelle le revêtement de polymère biodégradable éventuel contient un médicament thérapeutique.

3. Prothèse expansible selon la revendication 1 ou 2, dans laquelle le revêtement de polymère biodégradable contient un médicament thérapeutique choisi dans un groupe qui comprend les composés rapamycine, biolimus A9, évérolimus, zotarolimus, CRC-015, novolimus, tacrolimus et myolimus.

4. Prothèse expansible selon l'une quelconque des revendications précédentes, dans laquelle le revêtement de polymère éventuel contient un médicament thérapeutique choisi dans le groupe qui comprend des composés du paclitaxel et de dérivés de paclitaxel.

5. Prothèse expansible selon l'une quelconque des revendications précédentes, dans laquelle la vapeur de monomère est créée de préférence à partir d'au moins un des composés d'hexaméthyldisiloxane suivants, le divinyltétraméthyldisiloxane, le méthyltriméthoxysilane, le tétraméthylsiloxane, l'hexaméthyldisiloxane, le tétraméthylsilane et le tétraméthoxysilane.

6. Prothèse expansible selon la revendication 5, dans laquelle la couche organique biodégradable est une couche de polyméthylsiloxane uniforme appliquée par injection de vapeur d'HMDSO (hexaméthyldisiloxane) dans une chambre avec un plasma appliqué.

7. Prothèse expansible selon la revendication 5, dans laquelle la couche organique biodégradable est une couche de polyméthylsiloxane uniforme appliquée par injection de vapeur de DVTMDSO (divyltétraméthyldisiloxane) dans une chambre avec un plasma appliqué.

8. Prothèse expansible selon l'une quelconque des revendications précédentes, dans laquelle le métal biodégradable est du magnésium ou un alliage métallique de magnésium.

9. Prothèse expansible selon l'une quelconque des revendications précédentes, dans laquelle le revêtement de polymère éventuel est choisi dans un groupe qui comprend les composés cellulose, collagène, albumine, caséine, polysaccharides, polylactide (PLA), poly-L-lactide (PLLA), poly-d,l-lactide (PDLLA), polyglycol (PGA), polycaprolactone (PCL), poly-d,l-lectide-co-glycolide (PDLLA-PGA), acide polyhydroxybutirique (PHB), acide polyhydroxyvalérique (PHV), polyalkyl carbonates, poly ortho esters, polyéthylène téréphtalate (PET), acide polymalonique (PML), poly anhydrides, polyphosphazènes, poly aminoacides et leurs copolymères, acide hylauronique et des mélanges des composés précités.

10. Prothèse expansible selon l'une quelconque des revendications précédentes, où la prothèse est un stent et le médicament thérapeutique est contenu sur la surface de la couche de polymère biodégradable.

11. Prothèse expansible selon l'une quelconque des revendications précédentes, où la prothèse est un stent et le médicament thérapeutique est dispersé dans la couche de polymère biodégradable dans un rapport médicament sur polymère de 10 pour cent en poids à 70 pour cent en poids.

12. Prothèse expansible selon l'une quelconque des revendications précédentes, où la prothèse est un stent et le médicament thérapeutique est dispersé dans la couche de polymère contenant le médicament dans un rapport médicament sur polymère de 10 pour cent en poids à 70 pour cent en poids.

13. Procédé de production d'un stent biodégradable comprenant
le revêtement d'une structure métallique biodégradable tubulaire avec une couche organique biodégradable, et
le revêtement de la couche organique avec un revêtement de polymère biodégradable, la couche organique biodégradable étant une couche de siloxane.

14. Procédé selon la revendication 13, dans lequel la couche organique biodégradable est revêtue avec un revêtement de polymère contenant un médicament thérapeutique.

15. Procédé selon la revendication 13 ou 14, dans lequel le revêtement de la structure métallique biodégradable tubulaire avec une couche organique biodégradable comprend le revêtement de la structure métallique avec un composé de siloxane par un procédé de dépôt chimique en phase vapeur amélioré par un plasma.
